# EUROPEAN PATENT SPECIFICATION

(11) **EP 0 578 885 B1**
(45) Date of publication and mention of the grant of the patent: **16.12.1998**
(21) Application number: 92307101.3
(22) Date of filing: 04.08.1992
(51) Int. Cl.: A61L 27/00, A01N 1/02

(54) **Method of fixing biological tissue**
Verfahren zur Fixierung von biologischen Geweben
Procédé pour fixer un tissu biologique

(30) Priority: 15.07.1992 GB 9215002
(43) Date of publication of application: 19.01.1994
(73) Proprietor: NATIONAL UNIVERSITY OF SINGAPORE, Singapore 0511 (SG)
(72) Inventor: Khor, Eugene, Singapore 0511 (SG); Teoh, Swee Hin, Singapore 0511 (SG); Chian, Kerm Sin, 80400 Johor Bahru (MY); Loke, Weng Keong c/o Dr.E.Khor, Singapore 0511 (SG)
(74) Representative: King, James Bertram

(56) References cited:
- EP-A- 0 268 421
- WO-A-85/05274
- WO-A-87/02880
- WO-A-90/12055

## Description

Many materials, both synthetic and of biological origin, are used in medical devices. In surgically implanted prosthetic devices, these materials come in contact with human tissues giving rise to a variety of material-human tissue interactions *in vivo*. The success or failure of an implant device depends on how the material interacts with the human environment, which is governed by factors such as the origin of the material, the method of fabrication, the implant site and the duration of implantation.

Synthetic materials such as polymers are derived from chemicals and therefore the material properties are more readily reproducible and easier to handle. Furthermore, polymers may be suitably modified to adapt to the end application. However, because of their synthetic nature, incompatibility with the human tissue will invariably exist.

Material of biological origin must be fixed or tanned before it can be used. This is necessary to stabilise the tissue to degradation by forming cross links in the tissue. The established method for fixing biological tissue is the soaking of tissue in glutaraldehyde over several days. Glutaraldehyde, although successful, has been shown to give rise to a number of problems associated with host tissue reaction and slow degradation of the cross links *in vivo*.

Many recent reports addressing ways of improving on the glutaraldehyde process centres on the incorporation of anti-calcification agents. These agents are typically placed into the tissue, which with time will also be washed out. Another approach focuses on the utilisation of less toxic fixing reagents such as carbodiimide/glycerol. More recently, several patents have sought to incorporate anticalcification agents or polymers into biological tissue to improve on the viability of such biomaterials. These are based on the deposition of polymer solutions into tissue using diffusion mechanisms as the primary means of tissue/polymer interactions.

It is known from WO-A- 85 05274 to use diisocyanate in such fixing processes, see for example claims 5 and 6 and Examples 2 and 3. The polyisocyanates are said to increase the stability of the biological tissue. Additionally an aprotic solvent (acetone) is used in the Examples.

The present invention seeks also to improve on the glutaraldehyde process by replacing the glutaraldehyde fixing agent with a solution of prepolymer, polyetherpolyurethane. As a consequence, the advantages of the synthetic and biological materials are combined to produce a synergistic effect resulting in a biomaterial with enhanced durability.

An object of this invention is to provide a fixation process for biological tissue to enhance its durability and improve its anticalcification properties without changing drastically its mechanical properties. A prepolymer solution of polyetherpolyurethane may be used as the fixing agent. The polymer is chemically bonded to biological tissue to produce a biomaterial suitable for bioprosthesis applications.

The biological tissue fixed in this manner allows the incorporation of anticalcification or other advantageous agents such as pharmacological agents and anticoagulants to the biomaterial.

According to the invention there is provided a method of fixing biological tissue using a solution of polymer or a precursor of a polymer to produce a biomaterial with enhanced durability, characterised in that the polymer used is a polyurethane and that the method comprises treating biological tissue with an aprotic solvent to remove water from the tissue thereby rendering the tissue compatible for a reaction with a polyurethane or polyurethane precursor, reacting the dehydrated tissue with (a) a solution derived from diisocyanate to which a diol is subsequently added or with (b) a solution derived from a diisocyanate and a diol or with (c) an isocyanate terminated polyurethane solution or (d) a combination of two or more of the solutions (a), (b), (c), using an aprotic solvent as reaction solvent under inert and anhydrous conditions at a temperature up 60° Celsius, recovering the treated tissue and rinsing with aprotic solvent to remove excess polyetherpolyurethane solution, disinfecting the treated tissue, preferably by soaking it in a glutaraldehyde/formaldehyde mixture, and storing the treated tissue in a buffered saline solution preferably containing antifungal material. The reactant solvent may be used to remove excess polyurethane solution. Desirably the process is so carried out that the mechanical properties of the original tissue are maintained.

A wide range of polyurethanes may be used in the present invention including silicone modified polyurethanes.

Preferably an anticalcification agent and/or pharmacological agent and/or an anticoagulant is/are included before, during or after the fixation of the tissue.

The tissue may, for instance, be derived from pig, cow, sheep or kangaroo, such as animal tissue derived from heart valves or pericardial tissue. The tissue may be fully harvested animal tissue, glutaraldehyde or a carbodiimide/glycerol treated tissue, a collagen rich material or native collagen material that contains active cross linking sites.

Examples of suitable aprotic solvents are dimethyl sulphoxide, dimethylacetamide, dimethylformamide and tetrahydrofuran.

With this invention biological tissue can be fixed to give long term stability of fixed tissue.

Examples of biological materials that can be treated by the present process include animal tissue from various animals such as pig, cow, sheep or kangaroo of diverse origin, e.g. heart valves, pericardial tissue and other collagen high materials as well as reconstituted or native collagen materials that have potential cross linking sites. Tissue which has been previously fixed using chemical agents such as glutaraldehyde and/or carbodiimide/glycerol can also be subjected to the present process.

By way of example, fresh tissue may be harvested from an animal and immediately stored in buffered saline at 4° Celsius. The stored tissue may be subjected to the following exemplary procedures to fix them adequately to produce biomaterial comprising animal and synthetic character.

With reference to the following Examples 1,2 and 3 harvested tissue as described in the preceding paragraph is placed in dimethyl sulphoxide as aprotic solvent to dehydrate the tissue for up to 72 hours. The solvent may be changed periodically to quicken the dehydration process. After dehydration the tissue is subjected to the treatment described in any one of Examples 1 to 3.

### Example 1

In this example, the dehydrated tissue is stirred continuously in a solution of methylenediphenylene diisocyanate in anhydrous dimethyl sulphoxide [solution (a)]. The reaction is performed at a suitable temperature up to 60° Celsius, under dry N₂ or other inert gas. After a suitable time interval, for example 24 hours, a solution of tetramethylene ether glycol is introduced to complete the reaction which results in the formation of polyetherpolyurethane. Chain extenders typically used to form polyetherpolyurethanes and other dried compatible solvents can be introduced simultaneously. The treated tissue is removed, washed with reaction solvent briefly, disinfected and stored in buffered saline.

### Example 2

In this example, the reaction solution comprises methylenediphenylene diisocyanate and tetramethylene glycol in dimethyl sulphoxide [solution (b)]. Chain extenders typically used to form polyurethanes and other dried compatible solvents can also be introduced. Dehydrated tissue is then introduced into this reaction solution and is allowed to react while stirring is performed continuously. The reaction can be performed at a suitable temperature up to 60° Celsius but typically at 37° Celsius under dry N₂ or other inert gas. The treated tissue is removed, washed with reaction solvent briefly, disinfected and stored in buffered saline.

### Example 3

In this example, the prepolymer solution [solution (c)] comprises isocyanate terminated polyetherpolyurethane in dimethyl sulphoxide. The prepolymer is one which has been obtained by reaction of methylenediphenylene diisocyanate and tetramethylene glycol. Chain extenders typically used to form polyurethanes and other dried compatible solvents can also be introduced. Dehydrated tissue is then introduced into this prepolymer solution and is allowed to react while stirring is performed continuously. The reaction can be performed at a suitable temperature of up to 60° Celsius, but typically at 37° Celsius under dry N₂ or other inert gas. The treated tissue is removed, washed with reaction solvent briefly, disinfected and stored in buffered saline.

The preferred ratios of diisocyanate to diol in the examples is 2:1. Treated tissue can be disinfected in a formaldehyde/glutaraldehyde mixture. Tissue is preferably stored in buffered saline solution at neutral pH containing a small amount of suitable antifungal material.

One of the benefits of tissue fixed in this way is that it adopts characteristics of both synthetic and natural materials. Because fixation is achieved via a chemical bond formed between tissue and the polymeric fixing agent derived from polyetherpolyurethanes the tissue is expected to be more resistant to hydrolysis damage than glutaraldehyde.

The monomer or starting material can be functionalised for bonding with anticalcification or other advantageous agents such as pharmacological agents and anticoagulants. The incorporation of these agents can be achieved before, during or after the fixing process.

Glutaraldehyde fixing is known to shrink biological tissue considerably. The shrinkage of biological tissue in this invention can be minimised by controlling the reaction conditions.

A commercially available glutaraldehyde reagent for fixing biological tissue is a mixture of at least three components such as monomer, oligomer and polymer in varying quantities. This results in product variability when glutaraldehyde is used for fixing tissue. The procedure described herein is reproducible as the starting materials are substantially pure, and the polymeric constituents can be predetermined. Hence the tissue treated by the present method gives reproducible properties.

## Claims

1. A method of fixing biological tissue using a solution of polymer or a precursor of a polymer to produce a biomaterial with enhanced durability, characterised in that the polymer used is a polyurethane and that the method comprises treating biological tissue with an aprotic solvent to remove water from the tissue thereby rendering the tissue compatible for a reaction with a polyurethane or polyurethane precursor, reacting the dehydrated tissue with (a) a solution derived from diisocyanate to which a diol is subsequently added or with (b) a solution derived from a diisocyanate and a diol or with (c) an isocyanate terminated polyurethane solution or (d) a combination of two or more of the solutions (a), (b), (c), using an aprotic solvent as reaction solvent under inert and anhydrous conditions at a temperature up 60° Celsius, recovering the treated tissue and rinsing with aprotic solvent to remove excess polyetherpolyurethane solution, disinfecting the treated tissue and storing the treated tissue in a buffered saline solution.

2. A method according to Claim 1, wherein the treated tissue is disinfected by soaking it in a glutaraldehyde/formaldehyde mixture.

3. A method according to Claim 1 or Claim 2, wherein the treated tissue is stored in a buffered saline solution containing antifungal material.

4. A method according to any preceding claim, wherein the mechanical properties of the original tissue are maintained.

5. A method according to any preceding claim, wherein an anticalcification agent and/or pharmacological agent and/or an anticoagulant is/are included before, during or after the fixation of the tissue.

6. A method according to any preceding claim, wherein the tissue is freshly harvested animal tissue, glutaraldehyde or carbodiimide/glycerol treated tissue, a collagen rich material or a reconstituted or native collagen material that contains active cross linking sites.

7. A method according to any preceding claim, wherein the aprotic solvent is chosen from dimethyl sulphoxide, dimethylacetamide, dimethylformamide and tetrahydrofuran.

8. A method according to any preceding claim, which fixes biological tissue to give long term stability of fixed tissue.

9. A method according to any preceding claim, wherein the tissue is derived from pig, cow, sheep or kangaroo.

10. A method according to any preceding claim, wherein the tissue is animal tissue derived from heart valves or pericardial tissue.

## Patentansprüche

1. Verfahren zur Fixierung von biologischem Gewebe unter Verwendung einer Polymerlösung oder einem Vorläufer eines Polymers zur Herstellung eines Biomaterials erhöhter Haltbarkeit, **dadurch gekennzeichnet**, daß das Polymer ein Polyurethan ist und dabei ein biologisches Gewebe mit einem aprotischen Lösungsmittel zum Auswaschen des Wassers aus dem Gewebe behandelt wird, wodurch das Gewebe mit Polyurethan oder einem Vorläufer hiervon reagieren kann, daß das wasserfreie Gewebe zur chemischen Reaktion gebracht wird mit (a) einer Lösung aus Diisocyanat unter nachfolgender Zugabe eines Diols oder (b) einer Lösung aus einem Diisocyanat und einem Diol oder (c) einer Lösung aus einem Polyurethan mit endständigen Isocyanat Gruppen oder (d) einer Kombination von zwei oder mehreren Lösungen (a), (b), (c) unter Verwendung eines aprotischen Lösungsmittels als Reagenzlösung und unter inerten und wasserfreien Bedingungen bei einer Temperatur bis 60° C, daß das behandelte Gewebe entnommen und zum Auswaschen überschüssiger Polyetherpolyurethanlösung mit einem aprotischen Lösungsmittel gespült wird und daß das behandelte Gewebe desinfiziert und durch Behandlung in einer gepufferten Kochsalzlösung gelagert wird.

2. Verfahren nach Anspruch 1, **dadurch gekennzeichnet**, daß das behandelte Gewebe durch Tränkung in einem Glutaraldehyd/Formaldehyd-Gemisch desinfiziert wird.

3. Verfahren nach den Ansprüchen 1 oder 2, **dadurch gekennzeichnet**, daß das behandelte Gewebe in einer gepufferten Kochsalzlösung unter Zugabe eines Fungicids aufbewahrt wird.

4. Verfahren nach einem der vorhergehenden Ansprüche, **dadurch gekennzeichnet**, daß die mechanischen Eigenschaften des ursprünglichen Gewebes beibehalten werden.

5. Verfahren nach einem der vorhergehenden Ansprüche, **dadurch gekennzeichnet**, daß ein Mittel gegen Kalkbildung und/oder ein pharmakologisches Mittel und/oder ein Antikoagulant vor, während oder nach dem Fixierungsvorgang des Gewebes zugesetzt wird.

6. Verfahren nach einem der vorhergehenden Ansprüche, **dadurch gekennzeichnet**, daß das Gewebe ein frisch entnommenes tierisches Gewebe, ein mit Glutaraldehyd oder Cabodiimid/Glycerin behandeltes Gewebe, ein an Collagen reiches Material reiche Substanz oder ein rekonstituiertes oder natives Collagenmaterial mit aktiven Vernetzungsstellen ist.

7. Verfahren nach einem der vorhergehenden Ansprüche, **dadurch gekennzeichnet**, daß das aprotische Lösungsmittel Dimethylsufoxid, Dimethylacetamid, Demethylformamid und Tetrahydrofuran ist.

8. Verfahren nach einem der vorhergehenden Ansprüche **gekennzeichnet** durch eine Fixierung von biologischem Gewebe zwecks dessen Langzeitstabilisierung des fixierten Gewebes.

9. Verfahren nach einem der vorhergehenden Ansprüche, **dadurch gekennzeichnet**, daß das Gewebe von Schwein, Kuh, Schaf oder Känguruh stammt.

10. Verfahren nach einem der vorhergehenden Ansprüche, **dadurch gekennzeichnet**, daß das Gewebe ein tierisches Gewebe ist, das von Herzklappen oder Herzbeuteln stammt.

## Revendications

1. Procédé de fixation d'un tissu biologique utilisant une solution de polymère ou d'un précurseur d'un polymère pour produire une matière biologique de durabilité renforcée, caractérisé en ce que le polymère utilisé est un polyuréthanne et en ce qu'il comprend le traitement du tissu biologique avec un solvant aprotique pour éliminer l'eau de ce tissu de manière à le rendre compatible en vue d'une réaction avec un polyuréthanne ou un précurseur de polyuréthanne, la réaction du tissu déshydraté avec (a) une solution dérivée d'un diisocyanate à laquelle un diol est ensuite ajouté ou (b) une solution dérivée d'un diisocyanate et d'un diol ou (c) une solution de polyuréthanne à terminaison isocyanate ou (d) une combinaison de deux ou plus de deux des solutions (a) (b), (c), en utilisant un solvant aprotique comme solvant de réaction dans des conditions inertes et anhydres à une température s'élevant jusqu'à 60°C, l'isolement du tissu traité et son rinçage avec un solvant aprotique pour éliminer la solution de polyéther-polyuréthanne en excès, la désinfection du tissu traité et la conservation du tissu traité dans une solution saline tamponnée.

2. Procédé suivant la revendication 1, dans lequel le tissu traité est désinfecté par immersion de ce tissu dans un mélange glutaraldéhyde/formaldéhyde.

3. Procédé suivant la revendication 1 ou la revendication 2, dans lequel le tissu traité est conservé dans une solution tamponnée de sel contenant une matière antifongique.

4. Procédé suivant l'une quelconque des revendications précédentes, dans lequel les propriétés mécaniques du tissu originel sont conservées.

5. Procédé suivant l'une quelconque des revendications précédentes, dans lequel un agent anti-calcification et/ou un agent pharmacologique et/ou un anticoagulant est/sont inclus avant, pendant ou après la fixation du tissu.

6. Procédé suivant l'une quelconque des revendications précédentes, dans lequel le tissu est un tissu animal fraîchement prélevé, un tissu traité au glutaraldéhyde ou au mélange carbodiimide/glycérol, une matière riche en collagène ou une matière formée de collagène reconstitué ou naturel qui contient des sites actifs de réticulation.

7. Procédé suivant l'une quelconque des revendications précédentes, dans lequel le solvant aprotique est choisi entre le diméthylsulfoxyde le diméthylacétamide, le diméthylformamide et le tétrahydrofuranne.

8. Procédé suivant l'une quelconque des revendications précédentes, qui fixe un tissu biologique pour conférer au tissu fixé une stabilité à long terme.

9. Procédé suivant l'une quelconque des revendications précédentes, dans lequel le tissu provient de porc, de vache, de mouton ou de kangourou.

10. Procédé suivant l'une quelconque des revendications précédentes, dans lequel le tissu est un tissu animal dérivé de valvules cardiaques ou de tissu du péricarde.
